**Europäisches Patentamt**

**European Patent Office**

**Office européen des brevets**

(19)

(11) Veröffentlichungsnummer: **0 199 004**
**B1**

(12) # EUROPÄISCHE PATENTSCHRIFT

(45) Veröffentlichungstag der Patentschrift:
06.09.89

(21) Anmeldenummer: **86101637.6**

(22) Anmeldetag: **08.02.86**

(51) Int. Cl.⁴: **C07D 239/26, C09K 19/34**

(54) Pyrimidinderivate.

(30) Priorität: **23.02.85 DE 3506446**

(43) Veröffentlichungstag der Anmeldung:
**29.10.86 Patentblatt 86/44**

(45) Bekanntmachung des Hinweises auf die Patenterteilung:
**06.09.89 Patentblatt 89/36**

(84) Benannte Vertragsstaaten:
**CH DE FR GB LI NL**

(56) Entgegenhaltungen:
**EP-A- 0 025 119**
**EP-A- 0 084 194**
**EP-A- 0 104 011**
**EP-A- 0 152 808**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG, Frankfurter Strasse 250 Postfach 4119, D-6100 Darmstadt(DE)**

(72) Erfinder: **Bofinger, Klaus, Dr., Eberstädterstrasse 7, D-6109 Mühltal(DE)**
Erfinder: **Römer, Michael, Dr., Niederwiesenring 129a, D-6054 Rodgau 2(DE)**
Erfinder: **Scheuble, Bernhard, Dr., Am Grenzweg 18, D-6146 Alsbach(DE)**
Erfinder: **Weber, Georg, Wilhelm-Leuschner-Strasse 38, D-6106 Erzhausen(DE)**

**Beschreibung**

Die Erfindung betrifft Pyrimidinderivate der Formel I

R¹-Pyr-A¹-Z¹-A²-[Z²-A³]ₘ-R²   I

worin R¹ und R² jeweils eine Alkylgruppe mit 1 bis 12 C-Atomen, worin auch eine oder zwei nicht benachbarte CH₂-Gruppen durch -O-, -CO-, -O-CO-, -CO-O- oder -CH=CH- ersetzt sein können, einer der Reste R¹ und R² auch H, F, Cl, Br oder CN, Pyr Pyrimidin-2,5-diyl, A¹ und A³ jeweils trans-1,4-Cyclohexylen oder unsubstituiertes oder durch ein oder zwei F- und/oder Cl-Atome und/oder CH₃- und/oder CN-Gruppen substituiertes 1,4-Phenylen, A² unsubstituiertes oder durch ein oder zwei F- und/oder Cl-Atome und/oder CH₃- und/oder CN-Gruppen substituiertes 1,4-Phenylen,

Z¹ –CH₂CH₂–, –CH₂O–, oder –OCH₂–

Z² –CO-O-, –O-CO-, –CH₂CH₂–, –CH₂O–, –O-CH₂– oder eine Einfachbindung, und

m 0 oder 1 bedeuten.

Der Einfachheit halber bedeuten im folgenden Cy eine 1,4-Cyclohexylengruppe und Phe eine 1,4-Phenylengruppe, die gegebenenfalls auch durch ein oder zwei F- und/oder Cl-Atome und/oder CH₃- und/oder CN-Gruppen substituiert sein kann.

Ähnliche Verbindungen sind z.B. aus der DE-OS 3 040 632 bekannt. Die dort angegebenen Verbindungen enthalten jedoch im Gegensatz zu den vorliegenden keine Pyrimidin-2,5-diyl-Gruppen. In EP-A 25 119 werden Esterverbindungen vom Pyrimidintyp beschrieben.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplexraten herstellbar.

Überraschenderweise zeigte sich beim Zusatz von Verbindungen der Formel I zu flüssigkristallinen Phasen, daß eine erhebliche Verbesserung der Kennliniensteilheit der Mischung auftrat, so daß Verbindungen des Typs I als besonders vorteilhaft geeignete Substanzen zur Herstellung von flüssig-kristallinen Mischungen mit steiler Kennlinie anzusehen sind.

Sie ermöglichen damit die Entwicklung hoch multiplexbarer Mischungen, mit denen eine Drehzelle insbesondere im ersten Transmissionsminimum nach Gooch-Tarry betrieben werden kann. Damit ergibt sich eine sehr kleine Beobachtungswinkel-Abhängigkeit des Kontrastes.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielswiese die dielektrische und/oder optische Anisotropie einer solchen Phase zu senken oder um die elastischen Eigenschaften einer solchen Phase zu verbessern. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppe(n) und/oder C-C-Bindung(en) enthält, mit einem reduzierenden Mittel behandelt, oder daß man zur Herstellung von Ethern der Formel I (worin R¹ und/oder R² eine Alkylgruppe bedeutet, worin eine oder zwei nicht benachbarte CH₂-Gruppen durch O-Atome ersetzt sind und/oder Z eine -OCH₂- oder -CH₂-O-Gruppe ist) eine entsprechende Hydroxyverbindung verethert.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelement, insbesondere elektrooptische Anzeigeelemente, die derartige Phasen enthalten.

Die Verbindungen der Formel I umfassen dementsprechend Verbindungen der Teilformeln Ia und Ib (mit drei Ringen) sowie Ic und Id (mit vier Ringen):

R¹–Pyr–Phe–Z¹–Phe–R² Ia
R¹–Pyr–Cy–Z¹–Phe–R² Ib
R¹–Pyr–Phe–Z¹–Phe–Z²–A³–R² Ic
R¹–Pyr–Cy–Z¹–Phe–Z²–A³–R² Id

Darunter sind diejenigen der Teilformeln Ia und Ic bevorzugt.
Die bevorzugten Verbindungen der Teilformel Ia umfassen solche der Teilformeln Iaa bis Iac:

R¹–Pyr–Phe–OCH₂–Phe–R² Iaa
R¹–Pyr–Phe–CH₂CH₂–Phe–R² Iab
R¹–Pyr–Phe–CH₂O–Phe–R² Iac

Darunter sind diejenigen der Teilformel Iaa besonders bevorzugt.
Die bevorzugten Verbindungen der Teilformel Ib umfassen solche der Teilformeln Iba bis Ibc:

R¹–Pyr–Cy–OCH₂–Phe–R² Iba
R¹–Pyr–Cy–CH₂CH₂–Phe–R² Ibb
R¹–Pyr–Cy–CH₂O–Phe–R² Ibc

Bevorzugte Verbindungen der Teilformel Ic und Id sind diejenigen der Teilformeln Ica bis Icc:

R¹–Pyr–Phe–OCH₂–Phe–OCO–Cy–R² Ica
R¹–Pyr–Phe–OCH₂–Phe–OCO–Phe–R² Icb
R¹–Pyr–Phe–OCH₂–Phe–Cy–R² Icc

Darunter sind diejenigen der Teilformel Ica besonders bevorzugt.
In den Verbindungen der vor- und nachstehenden Formeln bedeuten R¹ und R² vorzugsweise Alkyl, ferner Alkoxy oder Oxaalkyl. Ferner bevorzugt sind Verbindungen der Formel I, worin R¹ eine der angegebenen bevorzugten Bedeutung hat (insbesondere Alkyl) und R² CN bedeutet.

A¹ ist bevorzugt Phe, insbesondere bevorzugt 1,4-Phenylen.
A² ist bevorzugt unsubstituiertes 1,4-Phenylen, ferner, im Falle m = 0, bevorzugt 2- bzw. 3-Fluor-1,4-Phenylen.
Z¹ ist vorzugsweise -OCH₂-.
m ist vorzugsweise 0.
Z² ist vorzugsweise eine Einfachbindung oder -O-CO-, insbesondere bevorzugt -O-CO-.
A³ ist vorzugsweise Cy.

Die Alkylreste in den Gruppen R¹ und/oder R² können geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl oder Dodecyl, ferner Methyl.
Falls R¹ und/oder R² Alkylreste bedeuten, in denen eine ("Alkoxy" bzw. "Oxaalkyl") oder zwei ("Alkoxyalkoxy" bzw. "Dioxaalkyl") CH₂-Gruppen durch O-Atome ersetzt sind, so können sie geradkettig oder verzweigt sein. Vorzugsweise sind sie geradkettig, haben 2, 3, 4, 5, 6 oder 7 C-Atome und bedeuten demnach bevorzugt Ethoxy, Propoxy, Butoxy, Pentoxy , Hexoxy, Heptoxy, 2-Oxapropyl (= Methoxymethyl), 2-, 3- oder 4-Oxapentyl, 2-, 3-, 4- oder 5-Oxahexyl, 2-, 3-, 4-, 5-, oder 6-Oxaheptyl, ferner Methoxy, Octoxy, Nonoxy, Decoxy, 2-, 3-, 4-, 5-, 6-, 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6-, oder 4,6-Dioxaheptyl.
Verbindungen der Formel I sowie der vor- und nachstehenden Teilformeln mit verzweigten Flügelgruppen R¹ bzw. R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy.
Unter den Verbindungen der Formel I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I7:

Alkyl-Pyr-Phe-OCH₂-Phe-Alkyl I1

Alkyl-Pyr-Phe-OCH₂-Phe-Alkoxy I2
Alkyl-Pyr-Phe-OCH₂-Phe-CN I3
Alkyl-Pyr-Phe-OCH₂-Phe-OCO-Cy-Alkyl I4
Alkyl-Pyr-Phe-OCH₂-Phe-OCO-Phe-Alkyl I5
Alkyl-Pyr-Phe-OCH₂-Phe-OCO-Phe-Alkoxy I6
Alkyl-Pyr-Phe-OCH₂-Phe-Cy-Alkyl I7

In den vorstehenden Formeln I1 bis I7 bedeutet Alkyl vorzugsweise eine geradkettige Alkylgruppe mit 2 bis 10 C-Atomen und Alkoxy eine geradkettige Alkoxygruppe mit 2 bis 12 C-Atomen. Weiterhin bevorzugt sind Verbindungen der Formel I1 bis I7, in denen an Stelle von Alkoxy eine 2-Oxaalkylgruppe mit 2 bis 12 C-Atomen vorhanden ist.

Unter Verbindungen der Formel I sind diejenigen Stereoisomeren bevorzugt, in denen die Cyclohexanringe trans-1,4-disubstituiert sind.

Die vorstehend genannten Formeln umschließen die beiden möglichen 2,5-Stellungsisomeren bezüglich der Pyrimidin-2,5-diyl-Gruppe. Vorzugsweise ist diese Gruppe in 5-Position mit R¹ verknüpft.

Besonders bevorzugt sind Verbindungen der Formel I sowie der vorstehenden Teilformeln, worin R¹ eine geradkettige Alkylgruppe mit 1 bis 10 C-Atomen und R² eine geradkettige Alkoxy-, Oxaalkyl- oder Alkylgruppe mit 2 bis 15 C-Atomen insbesondere mit 5 bis 12 C-Atomen bedeutet.

Besonders bevorzugt sind ferner Verbindungen der Formel I sowie der vorstehenden Teilformeln, worin R¹ oder R² eine Alkyl-, Alkoxy- oder Oxaalkylgruppe mit einem optisch aktiven Zentrum bedeutet.

Die Verbindungen der Formel I werden nach an sich bekannten Methoden hergestellt wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen (z. B. DE-OS 23 44 732, 24 50 088, 24 29 093, 25 02 904, 26 36 684, 27 01 591 und 27 52 957 betreffend Verbindungen mit 1,4-Cyclohexylen und 1,4-Phenylen-Gruppen; und DE-OS 32 01 721 betreffend Verbindungen mit -CH₂CH₂-Brückengliedern].

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atomes eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calicumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Ether der Formel I (worin R¹ und/oder R² eine Alkylgruppe bedeutet, worin eine oder zwei CH₂-Gruppen durch O-Atome ersetzt sind, und/oder worin Z¹ und/oder Z² eine -OCH₂- oder eine -CH₂O-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Verbindungen der Formel I mit optisch aktivem R¹ oder R² können völlig analog zu den aufgeführten Darstellungsmethoden hergestellt werden bei Verwendung der entsprechenden optisch aktiven Ausgangsprodukte.

Die erfindungsgemäßen flüssigkristallinen Phasen bestehen aus 2 bis 25, vorzugsweise 3 bis 15 Komponenten, darunter mindestestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Bis-cyclohexylethane, 1,2-Bis-phenylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren, R'-L-G-E-R" II worin L und E je ein carbo- oder heterocyclisches Ringsystem aus der aus 1,4-disubstituierten Benzol und Cyclohexanringen, 4,4'-disubstiuierten Biphenyl-, Phenylcyclohexan- und Cyclohexylcyclohexansystemen, 2,5-disubstituierten Pyrimidin- und 1,3-Dioxanringen, 2,6-disubstiuiertem Naphthalin, Di- und Tetrahydronaphthalin, Chinazolin und Tetrahydrochinazolin gebildeten Gruppe,

| G | –CH=CH– | –N(O)=– |
|---|---------|---------|
|   | –CH=CY– | –CH=N(O)– |
|   | –C≡C– | –CH$_2$–CH$_2$– |
|   | –CO–O– | –CH$_2$–O– |
|   | –CO–S– | –CH$_2$–S– |
|   | –CH=N– | –COO–Phe–COO– |

oder eine C-C-Einfachbindung, Y Halogen, vorzugsweise Chlor, oder -CN, und R' und R" Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, NO$_2$, CF$_3$, F, Cl oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle dieser Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika enthaltend 0,1 bis 40, vorzugsweise 0,5 bis 29 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53, 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturangaben sind in Grad Celsius angegeben. "Übliche Aufarbeitung" bedeutet: man gibt Wasser hinzu extrahiert mit Methylenchlorid, trennt ab, trocknet die organische Phase, dampft ein und reinigt das Produkt durch Kristallisation und/oder Chromatographie.

Beispiel 1

Eine Mischung von 10,2 g 2-p-Hydroxyphenyl-5-n-hexylpyrimidin, 8,6 g p-n-Propylbenzylbromid, 8,6 g Kaliumcarbonat und 50 ml Dimethylformamid wird 10 Stunden auf 90° erwärmt. Übliche Aufarbeitung liefert 4-(5-n-Hexylpyrimidin-2-yl)-phenyl-(p-n-propylbenzylether).

Analog werden hergestellt:

4-(5-Hexylpyrimidin-2-yl)-phenyl-(p-methylbenzylether)
4-(5-Hexylpyrimidin-2-yl)-phenyl-(p-ethylbenzylether)
4-(5-Hexylpyrimidin-2-yl)-phenyl-(p-butylbenzylether)
4-(5-Hexylpyrimidin-2-yl)-phenyl-(p-pentylbenzylether),
F. 72°, K. 104°, SIN 65 °, $\Delta\varepsilon$ =+1,0
4-(5-Hexylpyrimidin-2-yl)-phenyl-(p-hexylbenzylether),
F. 61°, K. 100,8 °, SIN 76 °, $\Delta n$ = 0,19
4-(5-Hexylpyrimidin-2-yl)-phenyl-(p-heptylbenzylether)
4-(5-Hexylpyrimidin-2-yl)-phenyl-(p-octylbenzylether)
4-(5-Hexylpyrimidin-2-yl)-phenyl-(p-nonylbenzylether)
4-(5-Hexylpyrimidin-2-yl)-phenyl-(p-decylbenzylether)
4-(5-Heptylpyrimidin-2-yl)-phenyl-(p-methylbenzylether)
4-(5-Heptylpyrimidin-2-yl)-phenyl-(p-ethylbenzylether)
4-(5-Heptylpyrimidin-2-yl)-phenyl-(p-propylbenzylether)
4-(5-Heptylpyrimidin-2-yl)-phenyl-(p-butylbenzylether)
4-(5-Heptylpyrimidin-2-yl)-phenyl-(p-pentylbenzylether)
F. 76°, K. 109°, SIN 86°, $\Delta\varepsilon$ = +1,0
4-(5-Heptylpyrimidin-2-yl)-phenyl-(p-hexylbenzylether),
F. 48°, K. 107°, SIN 92°, $\Delta n$ = 0,19
4-(5-Heptylpyrimidin-2-yl)-phenyl-(p-heptylbenzylether)
4-(5-Heptylpyrimidin-2-yl)-phenyl(p-octylbenzylether)
4-(5-Heptylpyrimidin-2-yl)-phenyl-(p-nonylbenzylether)
4-(5-Heptylpyrimidin-2-yl)-phenyl-(p-decylbenzylether)
4-(5-Heptylpyrimidin-2-yl)-phenyl-(p-cyanbenzylether)
4-(5-Heptylpyrimidin-2-yl)-phenyl-(p-methoxybenzylether)
4-(5-Heptylpyrimidin-2-yl)-phenyl-(p-ethoxybenzylether)
4-(5-Heptylpyrimidin-2-yl)-phenyl-(p-butoxybenzylether)
4-(5-Heptylpyrimidin-2-yl)-phenyl-(p-hexoxybenzylether)
4-(5-Heptylpyrimidin-2-yl)-phenyl-(p-octoxybenzylether)
4-(5-Octylpyrimidin-2-yl)-phenyl-(p-methylbenzylether)
4-(5-Octylpyrimidin-2-yl)-phenyl-(p-ethylbenzylether)
4-(5-Octylpyrimidin-2-yl)-phenyl-(p-propylbenzylether)
4-(5-Octylpyrimidin-2-yl)-phenyl-(p-butylbenzylether)
4-(5-Octylpyrimidin-2-yl)-phenyl-(p-pentylbenzylether)
4-(5-Octylpyrimidin-2-yl)-phenyl-(p-hexylbenzylether)
4-(5-Octylpyrimidin-2-yl)-phenyl-(p-heptylbenzylether)
4-(5-Octylpyrimidin-2-yl)-phenyl-(p-octylbenzylether)
4-(5-Octylpyrimidin-2-yl)-phenyl-(p-nonylbenzylether)
4-(5-Octylpyrimidin-2-yl)-phenyl-(p-decylbenzylether)
4-(5-Octylpyrimidin-2-yl)-phenyl-(p-cyanbenzylether)
4-(5-Octylpyrimidin-2-yl)-phenyl-(p-methoxybenzylether)
4-(5-Octylpyrimidin-2-yl)-phenyl-(p-ethoxybenzylether)
4-(5-Octylpyrimidin-2-yl)-phenyl-(p-butoxybenzylether)
4-(5-Octylpyrimidin-2-yl)-phenyl-(p-hexoybenzylether)
4-(5-Octylpyrimidin-2-yl)-phenyl-(p-octoxybenzylether)
4-(5-Nonylpyrimidin-2-yl)-phenyl-(p-methylbenzylether)
4-(5-Nonylpyrimidin-2-yl)-phenyl-(p-ethylbenzylether)
4-(5-Nonylpyrimidin-2-yl)-phenyl-(p-propylbenzylether)
4-(5-Nonylpyrimidin-2-yl)-phenyl-(p-butylbenzylether)
4-(5-Nonylpyrimidin-2-yl)-phenyl-(p-pentylbenzylether)
F. 52°, K. 113°, SIN 107°, $\Delta\varepsilon$ = + 0,9
4-(5-Nonylpyrimidin-2-yl)-phenyl-(p-hexylbenzylether)
F. 66°, K. 110°, SIN 109°, $\Delta n$ = 0,18
4-(5-Nonylpyrimidin-2-yl)-phenyl-(p-heptylbenzylether),F.51°,K.112°
4-(5-Nonylpyrimidin-2-yl)-phenyl-(p-octylbenzylether)
4-(5-Nonylpyrimidin-2-yl)-phenyl-(p-nonylbenzylether)
4-(5-Nonylpyrimidin-2-yl)-phenyl-(p-decylbenzylether)
4-(5-Nonylpyrimidin-2-yl)-phenyl-(p-cyanbenzylether),F.105°,K.168°
4-(5-Nonylpyrimidin-2-yl)-phenyl-(p-methoxybenzylether)
4-(5-Nonylpyrimidin-2-yl)-phenyl-(p-ethoxybenzylether)
4-(5-Nonylpyrimidin-2-yl)-phenyl-(p-butoxybenzylether)
4-(5-Nonylpyrimidin-2-yl)-phenyl-(p-hexoxybenzylether)
4-(5-Nonylpyrimidin-2-yl)-phenyl-(p-octoxybenzylether)
4-(5-Decylpyrimidin-2-yl)-phenyl-(p-methylbenzylether)

4-(5-Decylpyrimidin-2-yl)-phenyl-(p-ethylbenzylether)
4-(5-Decylpyrimidin-2-yl)-phenyl-(p-propylbenzylether)
4-(5-Decylpyrimidin-2-yl)-phenyl-(p-butylbenzylether)
4-(5-Decylpyrimidin-2-yl)-phenyl-(p-pentylbenzylether)
4-(5-Decylpyrimidin-2-yl)-phenyl-(p-hexylbenzylether)
4-(5-Decylpyrimidin-2-yl)-phenyl-(p-heptylbenzylether)
4-(5-Decylpyrimidin-2-yl)-phenyl-(p-octylbenzylether)
4-(5-Decylpyrimidin-2-yl)-phenyl-(p-nonylbenzylether)
4-(5-Decylpyrimidin-2-yl)-phenyl-(p-decylbenzylether)
4-(5-Decylpyrimidin-2-yl)-phenyl-(p-cyanbenzylether)
4-(5-Decylpyrimidin-2-yl)-phenyl-(p-methoxybenzylether)
4-(5-Decylpyrimidin-2-yl)-phenyl-(p-ethoxybenzylether)
4-(5-Decylpyrimidin-2-yl)-phenyl-(p-butoxybenzylether)
4-(5-Decylpyrimidin-2-yl)-phenyl-(p-hexoybenzylether)
4-(5-Decylpyrimidin-2-yl)-phenyl-(p-octoxybenzylether)
4-(5-Pentylpyrimidin-2-yl)-phenyl-(p-methylbenzylether)
4-(5-Pentylpyrimidin-2-yl)-phenyl-(p-ethylbenzylether)
4-(5-Pentylpyrimidin-2-yl)-phenyl-(p-propylbenzylether)
4-(5-Pentylpyrimidin-2-yl)-phenyl-(p-butylbenzylether)
4-(5-Pentylpyrimidin-2-yl)-phenyl-(p-pentylbenzylether)
4-(5-Pentylpyrimidin-2-yl)-phenyl-(p-hexylbenzylether)
4-(5-Pentylpyrimidin-2-yl)-phenyl-(p-heptylbenzylether)
4-(5-Pentylpyrimidin-2-yl)-phenyl-(p-octylbenzylether)
4-(5-Pentylpyrimidin-2-yl)-phenyl-(p-nonylbenzylether)
4-(5-Pentylpyrimidin-2-yl)-phenyl-(p-decylbenzylether)
4-(5-Pentylpyrimidin-2-yl)-phenyl-(p-cyanbenzylether)
4-(5-Pentylpyrimidin-2-yl)-phenyl-(p-methoxybenzylether)
4-(5-Pentylpyrimidin-2-yl)-phenyl-(p-ethoxybenzylether)
4-(5-Pentylpyrimidin-2-yl)-phenyl-(p-butoxybenzylether)
4-(5-Pentylpyrimidin-2-yl)-phenyl-(p-hexoxybenzylether)
4-(5-Pentylpyrimidin-2-yl)-phenyl-(p-octoxybenzylether)
4-(5-Butylpyrimidin-2-yl)-phenyl-(p-methylbenzylether)
4-(5-Butylpyrimidin-2-yl)-phenyl-(p-ethylbenzylether)
4-(5-Butylpyrimidin-2-yl)-phenyl-(p-propylbenzylether)
4-(5-Butylpyrimidin-2-yl)-phenyl-(p-butylbenzylether)
4-(5-Butylpyrimidin-2-yl)-phenyl-(p-pentylbenzylether)
4-(5-Butylpyrimidin-2-yl)-phenyl-(p-hexylbenzylether)
4-(5-Butylpyrimidin-2-yl)-phenyl-(p-heptylbenzylether)
4-(5-Butylpyrimidin-2-yl)-phenyl-(p-octylbenzylether)
4-(5-Butylpyrimidin-2-yl)-phenyl-(p-nonylbenzylether)
4-(5-Butylpyrimidin-2-yl)-phenyl-(p-decylbenzylether)
4-(5-Butylpyrimidin-2-yl)-phenyl-(p-cyanbenzylether)
4-(5-Butylpyrimidin-2-yl)-phenyl-(p-methoxybenzylether)
4-(5-Butylpyrimidin-2-yl)-phenyl-(p-ethoxybenzylether)
4-(5-Butylpyrimidin-2-yl)-phenyl-(p-butoxybenzylether)
4-(5-Butylpyrimidin-2-yl)-phenyl-(p-hexoybenzylether)
4-(5-Butylpyrimidin-2-yl)-phenyl-(p-octoxybenzylether)
4-(5-Propylpyrimidin-2-yl)-phenyl-(p-methylbenzylether)
4-(5-Propylpyrimidin-2-yl)-phenyl-(p-ethylbenzylether)
4-(5-Propylpyrimidin-2-yl)-phenyl-(p-propylbenzylether)
4-(5-Propylpyrimidin-2-yl)-phenyl-(p-butylbenzylether)
4-(5-Propylpyrimidin-2-yl)-phenyl-(p-pentylbenzylether)
4-(5-Propylpyrimidin-2-yl)-phenyl-(p-hexylbenzylether)
4-(5-Propylpyrimidin-2-yl)-phenyl-(p-heptylbenzylether)
4-(5-Propylpyrimidin-2-yl)-phenyl-(p-octylbenzylether)
4-(5-Propylpyrimidin-2-yl)-phenyl-(p-nonylbenzylether)
4-(5-Propylpyrimidin-2-yl)-phenyl-(p-decylbenzylether)
4-(5-Propylpyrimidin-2-yl)-phenyl-(p-cyanbenzylether)
4-(5-Propylpyrimidin-2-yl)-phenyl-(p-methoxybenzylether)
4-(5-Propylpyrimidin-2-yl)-phenyl-(p-ethoxybenzylether)
4-(5-Propylpyrimidin-2-yl)-phenyl-(p-butoxybenzylether)
4-(5-Propylpyrimidin-2-yl)-phenyl-(p-hexoxybenzylether)
4-(5-Propylpyrimidin-2-yl)-phenyl-(p-octoxybenzylether)

Beispiel 2

Zu einem Gemisch von 17,8 g p-n-Hexylphenol und 37 g trans-4-trans-(5-n-Hexylpyrimidin-2-yl)-cyclohexylmethanol-mesylat [erhältlich aus dem entsprechenden Alkohol, welcher durch Umsetzung von 2-n-Hexyl-propan-1,3-dialdehyd-tetraethyl-acetal mit trans-4-Cyancyclohexylmethanol bzw. dem entsprechenden Amidin nach literaturbekannten Methoden zugänglich ist] in 200 ml Toluol gibt man 5 g Tetrabutylammonium-hydrogensulfat und 20 ml 50 %ige NaOH, erhitzt das Gemisch drei Stunden unter Rühren auf 70 °, arbeitet wie üblich auf und erhält trans-4-(5-n-Hexylpyrimidin-2-yl)-cyclohexylmethyl-(p-n-hexyl-phenyl)-ether.

Analog werden hergestellt:

trans-4-(5-Hexylpyrimidin-2-yl)-cyclohexylmethyl-(p-propyl-phenyl)-ether
trans-4-(5-Hexylpyrimidin-2-yl)-cyclohexylmethyl-(p-butyl phenyl)-ether
trans-4-(5-Hexylpyrimidin-2-yl)-cyclohexylmethyl-(p-pentyl phenyl)-ether
trans-4-(5-Hexylpyrimidin-2-yl)-cyclohexylmethyl-(p-heptyl phenyl)-ether
trans-4-(5-Hexylpyrimidin-2-yl)-cyclohexylmethyl-(p-nonyl phenyl)-ether
trans-4-(5-Hexylpyrimidin-2-yl)-cyclohexylmethyl-(p-decyl phenyl)-ether
trans-4-(5-Pentylpyrimidin-2-yl)-cyclohexylmethyl-(p-propyl-phenyl)-ether
trans-4-(5-Pentylpyrimidin-2-yl)-cyclohexylmethyl-(p-butyl-phenyl)-ether
trans-4-(5-Pentylpyrimidin-2-yl)-cyclohexylmethyl-(p-pentyl-phenyl)-ether
trans-4-(5-Pentylpyrimidin-2-yl)-cyclohexylmethyl-(p-hexyl-phenyl)-ether
trans-4-(5-Pentylpyrimidin-2-yl)-cyclohexylmethyl-(p-heptyl-phenyl)-ether
trans-4-(5-Pentylpyrimidin-2-yl)-cyclohexylmethyl-(p-octyl-phenyl)-ether
trans-4-(5-Pentylpyrimidin-2-yl)-cyclohexylmethyl-(p-nonyl-phenyl)-ether
trans-4-(5-Pentylpyrimidin-2-yl)-cyclohexylmethyl-(p-decyl-phenyl)-ether
trans-4-(5-Pentylpyrimidin-2-yl)-cyclohexylmethyl-(p-dodecyl-phenyl)-ether
trans-4-(5-Pentylpyrimidin-2-yl)-cyclohexylmethyl-(p-cyan-phenyl)-ether
trans-4-(5-Pentylpyrimidin-2-yl)-cyclohexylmethyl-(p-cyan-m-fluorphenyl)-ether
trans-4-(5-Heptylpyrimidin-2-yl)-cyclohexylmethyl-(p-propyl-phenyl)-ether
trans-4-(5-Heptylpyrimidin-2-yl)-cyclohexylmethyl-(p-butyl-phenyl)-ether
trans-4-(5-Heptylpyrimidin-2-yl)-cyclohexylmethyl-(p-pentyl-phenyl)-ether
trans-4-(5-Heptylpyrimidin-2-yl)-cyclohexylmethyl-(p-hexyl-phenyl)-ether
trans-4-(5-Heptylpyrimidin-2-yl)-cyclohexylmethyl-(p-heptyl-phenyl)-ether
trans-4-(5-Heptylpyrimidin-2-yl)-cyclohexylmethyl-(p-octyl-phenyl)-ether
trans-4-(5-Heptylpyrimidin-2-yl)-cyclohexylmethyl-(p-nonyl-phenyl)-ether
trans-4-(5-Heptylpyrimidin-2-yl)-cyclohexylmethyl-(p-decyl-phenyl)-ether
trans-4-(5-Heptylpyrimidin-2-yl)-cyclohexylmethyl-(p-dodecyl-phenyl)-ether
trans-4-(5-Heptylpyrimidin-2-yl)-cyclohexylmethyl-(p-cyan-phenyl)-ether
trans-4-(5-Heptylpyrimidin-2-yl)-cyclohexylmethyl-(p-cyan-m-fluorphenyl)-ether
trans-4-(5-Nonylpyrimidin-2-yl)-cyclohexylmethyl-(p-propyl-phenyl)-ether
trans-4-(5-Nonylpyrimidin-2-yl)-cyclohexylmethyl-(p-butyl-phenyl)-ether
trans-4-(5-Nonylpyrimidin-2-yl)-cyclohexylmethyl-(p-pentyl-phenyl)-ether
trans-4-(5-Nonylpyrimidin-2-yl)-cyclohexylmethyl-(p-hexyl-phenyl)-ether
trans-4-(5-Nonylpyrimidin-2-yl)-cyclohexylmethyl-(p-heptyl-phenyl)-ether
trans-4-(5-Nonylpyrimidin-2-yl)-cyclohexylmethyl-(p-octyl-phenyl)-ether
trans-4-(5-Nonylpyrimidin-2-yl)-cyclohexylmethyl-(p-nonyl-phenyl)-ether
trans-4-(5-Nonylpyrimidin-2-yl)-cyclohexylmethyl-(p-decyl-phenyl)-ether
trans-4-(5-Nonylpyrimidin-2-yl)-cyclohexylmethyl-(p-dodecyl-phenyl)-ether
trans-4-(5-Nonylpyrimidin-2-yl)-cyclohexylmethyl-(p-cyan-phenyl)-ether
trans-4-(5-Nonylpyrimidin-2-yl)-cyclohexylmethyl-(p-cyan-m-fluorphenyl)-ether
trans-4-(5-Decylpyrimidin-2-yl)-cyclohexylmethyl-(p-propyl-phenyl)-ether
trans-4-(5-Decylpyrimidin-2-yl)-cyclohexylmethyl-(p-butyl-phenyl)-ether
trans-4-(5-Decylpyrimidin-2-yl)-cyclohexylmethyl-(p-pentyl-phenyl)-ether
trans-4-(5-Decylpyrimidin-2-yl)-cyclohexylmethyl-(p-hexyl-phenyl)-ether
trans-4-(5-Decylpyrimidin-2-yl)-cyclohexylmethyl-(p-heptyl-phenyl)-ether
trans-4-(5-Decylpyrimidin-2-yl)-cyclohexylmethyl-(p-octyl-phenyl)-ether
trans-4-(5-Decylpyrimidin-2-yl)-cyclohexylmethyl-(p-nonyl-phenyl)-ether
trans-4-(5-Decylpyrimidin-2-yl)-cyclohexylmethyl-(p-decyl-phenyl)-ether
trans-4-(5-Decylpyrimidin-2-yl)-cyclohexylmethyl-(p-dodecyl-phenyl)-ether
trans-4-(5-Decylpyrimidin-2-yl)-cyclohexylmethyl-(p-cyan-phenyl)-ether
trans-4-(5-Decylpyrimidin-2-yl)-cyclohexylmethyl-(p-cyan-m-fluorphenyl)-ether
trans-4-(5-Dodecylpyrimidin-2-yl)-cyclohexylmethyl-(p-propyl-phenyl)-ether
trans-4-(5-Dodecylpyrimidin-2-yl)-cyclohexylmethyl-(p-butyl-phenyl)-ether
trans-4-(5-Dodecylpyrimidin-2-yl)-cyclohexylmethyl-(p-pentyl-phenyl)-ether
trans-4-(5-Dodecylpyrimidin-2-yl)-cyclohexylmethyl-(p-hexyl-phenyl)-ether

trans-4-(5-Dodecylpyrimidin-2-yl)-cyclohexylmethyl-(p-heptyl-phenyl)-ether
trans-4-(5-Dodecylpyrimidin-2-yl)-cyclohexylmethyl-(p-octyl-phenyl)-ether
trans-4-(5-Dodecylpyrimidin-2-yl)-cyclohexylmethyl-(p-nonyl-phenyl)-ether
trans-4-(5-Dodecylpyrimidin-2-yl)-cyclohexylmethyl-(p-decyl-phenyl)-ether
trans-4-(5-Dodecylpyrimidin-2-yl)-cyclohexylmethyl-(p-dodecyl-phenyl)-ether
trans-4-(5-Dodecylpyrimidin-2-yl)-cyclohexylmethyl-(p-cyan-phenyl)-ether
trans-4-(5-Dodecylpyrimidin-2-yl)-cyclohexylmethyl-(p-cyan-m-fluorphenyl)-ether

Beispiel 3

Ein Gemisch von 0,04 M p-(5-n-Heptalpyrimidin-2-yl)-benzylbromid [erhältlich aus dem entsprechenden Benzyl-alkohol, welcher durch Umsetzung von 2-n-Heptyl-propan-1,3-dialdehydtetraethylacetal mit p-Hydroxymethylbenzonitril bzw. dem entsprechenden Amidin nach literaturbe kannten Methoden zugänglich ist], 0,04 M 4-Cyan-3-fluorphenol, 0,2 M Kaliumcarbonat und 150 ml 2-Butanon wird 48 Stunden gekocht, abgekühlt und wie üblich aufgearbeitet. Man erhält p-(5-n-Heptylpyrimidin-2-yl)-benzyl-(4-cyan-3-fluorphenyl)-ether.
Analog werden hergestellt

p-(5-Propylpyrimidin-2-yl)-benzyl-(4-cyan-3-fluorphenyl)-ether
p-(5-Butylpyrimidin-2-yl)-benzyl-(4-cyan-3-fluorphenyl)-ether
p-(5-Pentylpyrimidin-2-yl)-benzyl-(4-cyan-3-fluorphenyl)-ether
p-(5-Hexylpyrimidin-2-yl)-benzyl-(4-cyan-3-fluorphenyl)-ether
p-(5-Octylpyrimidin-2-yl)-benzyl-(4-cyan-3-fluorphenyl)-ether
p-(5-Nonylpyrimidin-2-yl)-benzyl-(4-cyan-3-fluorphenyl)-ether
p-(5-Decylpyrimidin-2-yl)-benzyl-(4-cyan-3-fluorphenyl)-ether
p-(5-Dodecylpyrimidin-2-yl)-benzyl-(4-cyan-3-fluorphenyl)-ether

Beispiel 4

Analog Beispiel 1 erhält man aus den entsprechenden optisch aktiven Ausgangsprodukten optisch aktive Verbindungen der Formel 1: Eine Mischung von 11,9 g 2-p-Hydroxyphenyl-5-n-nonylpyrimidin, 10,2 g p-(2-Methylbutoxy)-benzylbromid (herstellbar aus 4-Hydroxybenzaldehyd und 2-Methylbutyl-mesylat in Gegenwart von Kaliumcarbonat in DMF, anschließend Reduktion des Benzaldehyds zum Benzylalko-hol und Umsetzung mit PBr3 zum entsprechenden Benzylbromid), 8,6 g Kaliumcarbonat und 50 ml Dimethylformamid wird 10 Stunden auf 90° erwärmt. Übliche Aufarbeitung liefert optisch aktives 4-(5-n-Nonylpyrimidin-2-yl)-phenyl-[p-(2-methylbutoxy)benzylether], S-Ch 103°, Ch-I 105°.
Analog werden hergestellt:

4-(5-Hexylpyrimidin-2-yl)-phenyl-[p-(2-methylbutoxy)-benzylether]
4-(5-Heptylpyrimidin-2-yl)-phenyl-[p-(2-methylbutoxy)-benzylether]
4-(5-Octylpyrimidin-2-yl)-phenyl-[p-(2-methylbutoxy)-benzylether]
4-(5-Decylpyrimidin-2-yl)-phenyl-[p-(2-methylbutoxy)-benzylether]
4-(5-Pentylpyrimidin-2-yl)-phenyl-[p-(2-methylbutoxy)-benzylether]
4-(5-Butylpyrimidin-2-yl)-phenyl-[p-(2-methylbutoxy)-benzylether]
4-(5-Propylpyrimidin-2-yl)-phenyl-[2-(2-methylbutoxy)-benzylether]

Beispiel 5

Analog Beispiel 4 erhält man aus 2-p-Hydroxyphenyl-5-n-nonylpyrimidin und p-Brommethylbenzoesäure-2-methyl-butylester (darstellbar durch azeotrope Veresterung der p-Brommethylbenzoesäure mit 2-Methyl-1-butanol) 4-(5-n-Nonylpyrimidin-2-yl)-phenyl-[p-(2-methylbutoxy-carbonyl)-benzy-lether], F. 29°.
Analog werden hergestellt:

4-(5-Hexylpyrimidin-2-yl)-phenyl-[p-(2-methylbutoxy-carbonyl)-benzylether]
4-(5-Heptylpyrimidin-2-yl)-phenyl-[p-(2-methylbutoxy-carbonyl)-benzylether]
4-(5-Octylpyrimidin-2-yl)-phenyl-[p-(2-methylbutoxy-carbonyl)-benzylether]
4-(5-Decylpyrimidin-2-yl)-phenyl-[p-(2-methylbutoxy-carbonyl)-benzylether]
4-(5-Pentylpyrimidin-2-yl)-phenyl-[p-(2-methylbutoxy-carbonyl)-benzylether]
4-(5-Butylpyrimidin-2-yl)-phenyl-[p-(2-methylbutoxy-carbonyl)-benzylether]
4-(5-Propylpyrimidin-2-yl)-phenyl-[p-(2-methylbutoxy-carbonyl)-benzylether]

Die folgenden Beispiele betreffen erfindungsgemäße flüssigkristalline Phasen:

<u>Beispiel A</u>

Eine flüssigkristalline Phase bestehend aus

5 % 2-p-Cyanphenyl-5-ethyl-1,3-dioxan,
8 % 2-p-Cyanphenyl-5-propyl-1,3-dioxan,
8 % 2-p-Cyanphenyl-5-butyl-1,3-dioxan,
5 % 2-p-Cyanphenyl-5-pentyl-1,3-dioxan,
5 % 4-(5-Hexylpyrimidin-2-yl)-phenyl-(p-pentylbenzyl)-ether,
12 % 4-(5-Hexylpyrimidin-2-yl)-phenyl-(p-hexylbenzyl)-ether,
11 % 4-(5-Heptylpyrimidin-2-yl)-phenyl-(p-pentylbenzyl)-ether,
5 % 4,4'-Bis-(trans-4-pentylcyclohexyl)-biphenyl,
11 % trans-1-p-Methoxyphenyl-4-propylcyclohexan,
5 % 2-p-Methoxyphenyl-5-hexylpyrimidin,
5 % 2-p-Pentyloxyphenyl-5-hexylpyrimidin,
5 % 2-p-Heptyloxyphenyl-5-hexylpyrimidin,
5 % 2-p-Methoxyphenyl-5-heptylpyrimidin,
5 % 2-p-Heptyloxyphenyl-5-heptylpyrimidin und
5 % 2-p-Nonyloxyphenyl-5-heptylpyrimidin
hat einen Schmelzpunkt von -10° und einen Klärpunkt von 56°.

<u>Beispiel B</u>

Eine flüssigkristalline Phase bestehend aus

5 % 2-p-Cyanphenyl-5-ethyl-1,3-dioxan,
7 % 2-p-Cyanphenyl-5-propyl-1,3-dioxan,
8 % 2-p-Cyanphenyl-5-butyl-1,3-dioxan,
7 % 2-p-Cyanphenyl-5-pentyl-1,3-dioxan,
5 % 4-(5-Hexylpyrimidin-2-yl)-phenyl-(p-pentylbenzyl)-ether,
10 % 4-(5-Heptylpyrimidin-2-yl)-phenyl-(p-pentylbenzyl)-ether,
8 % 4-(5-Heptylpyrimidin-2-yl)-phenyl-(p-hexylbenzyl)-ether,
5 % 4-(trans-4-Pentylcyclohexyl)-4'-(trans-4-propyl-cyclohexyl)-2-fluorbiphenyl,
5 % 4,4'-Bis-(trans-4-pentylcyclohexyl)-2-fluorbiphenyl,
10 % trans-1-p-Methoxyphenyl-4-propylcyclohexan,
5 % 2-p-Methoxyphenyl-5-hexylpyrimidin,
5 % 2-p-Pentyloxyphenyl-5-hexylpyrimidin,
5 % 2-p-Heptyloxyphenyl-5-hexylpyrimidin,
5 % 2-p-Methoxyphenyl-5-heptylpyrimidin,
5 % 2-p-Heptyloxyphenyl-5-heptylpyrimidin und
5 % 2-p-Nonyloxyphenyl-5-heptylpyrimidin
hat einen Schmelzpumkt von -14°, einen Klärpunkt von 65° und eine relativ hohe optische Anisotropie.

<u>Beispiel C</u>

Eine flüssigkristalline Phase bestehend aus

8 % 4-Butyl-4'Cyanbiphenyl,
11 % 4-Pentyl-4'Cyanbiphenyl,
8 % 4-Hexyl-4'Cyanbiphenyl,
5 % 4-(5-Hexylpyrimidin-2-yl)-phenyl-(p-pentylbenzyl)-ether,
10 % 4-(5-Heptylpyrimidin-2-yl)-phenyl-(p-pentylbenzyl)-ether,
8 % 4-(5-Heptylpyrimidin-2-yl)-phenyl-(p-hexylbenzyl)-ether,
5 % 4-(trans-4-Pentylcyclohexyl)-4'-(trans-4-propyl-cyclohexyl)-2-fluorbiphenyl,
5 % 4,4'-Bis-(trans-4-pentylcyclohexyl)-2-fluorbiphenyl,
10 % trans-1-p-Methoxyphenyl-4-propylcyclohexan,
5 % 2-p-Methoxyphenyl-5-hexylpyrimidin,
5 % 2-p-Pentyloxyphenyl-5-hexylpyrimidin,
5 % 2-p-Heptyloxyphenyl-5-hexylpyrimidin,
5 % 2-p-Methoxyphenyl-5-heptylpyrimidin,
5 % 2-p-Heptyloxyphenyl-5-heptylpyrimidin und
5 % 2-p-Nonyloxyphenyl-5-heptylpyrimidin
hat einen Schmelzpunkt von -13°, einen Klärpunkt von 63° und eine relativ hohe optische Anisotropie.

Beispiel D

Eine flüssigkristalline Phase bestehend aus

5 % 2-p-Cyanphenyl-5-pentylpyrimidin,
10 % 2-p-Cyanphenyl-5-heptylpyrimidin,
6 % 2-p-Cyanphenyl-5-(p-butylphenyl)-pyrimidin,
6 % 4-(5-Hexylpyrimidin-2-yl)-phenyl -(p-pentylbenzyl)-ether,
13 % 4-(5-Heptylpyrimidin-2-yl)-phenyl -(p-pentylbenzyl)-ether,
10 % 4-(5-Heptylpyrimidin-2-yl)-phenyl -(p-hexylbenzyl)-ether,
5 % 4-(trans-4-Pentylcyclohexyl)-4'-(trans-4-propyl-cyclohexyl)-2-fluorbiphenyl,
5 % 4,4'-Bis-(trans-4-pentylcyclohexyl)-2-fluorbiphenyl,
10 % trans-1-p-Methoxyphenyl-4-propylcyclohexan,
5 % 2-p-Methoxyphenyl-5-hexylpyrimidin,
5 % 2-p-Pentyloxyphenyl-5-hexylpyrimidin,
5 % 2-p-Heptyloxyphenyl-5-hexylpyrimidin,
5 % 2-p-Methoxyphenyl-5-heptylpyrimidin,
5 % 2-p-Heptyloxyphenyl-5-heptylpyrimidin und
5 % 2-p-Nonyloxyphenyl-5-heptylpyrimidin

hat einen Schmelzpunkt von -5°, einen Klärpunkt von 85°, eine relativ hohe optische Anisotropie und eine besonders niedrige Schwellenspannung.

Beispiel E

Man stellt eine flüssigkristalline Phase her aus

17 % p-trans-4-Propylcyclohexyl-benzonitril,
23 % p-trans-4-Pentylcyclohexyl-benzonitril,
16 % trans-1-p-Ethoxyphenyl-4-propylcyclohexan,
14 % trans-1-p-Butoxyphenyl-4-propylcyclohexan,
10 % 4-(trans-4-Pentylcyclohexyl)-4'-(trans-4-propyl-cyclohexyl)-biphenyl,
10 % 2-(4-Ethoxyphenyl)-5-(trans-4-propylcyclohexyl)-pyrimidin und
10 % 4-(5-Heptylpyrimidin-2-yl)-phenyl-(p-hexylbenzyl-ether).

Beispiel F

Man stellt eine flüssigkristalline Phase her aus

21 % p-trans-4-Ethylcyclohexyl-benzonitril,
22 % p-trans-4-Butylcyclohexyl-benzonitril,
14 % 4-Ethyl-4'-cyanbiphenyl,
18 % 4-Butyl-4-cyanbiphenyl,
10 % 2-(trans-4-Propylcyclohexyl)-pyrimidin-5-carbonsäure-(p-pentylphenylester),
10 % 4-(5-Hexylpyrimidin-2-yl)-phenyl-(p-pentylbenzyl-ether) und
5 % 4-(5-Heptylpyrimidin-2-yl)-phenyl-(p-hexylbenzyl-ether).

## Patentansprüche

1. Pyrimidinderivate der Formel I
$R^1$–Pyr–$A^1$–$Z^1$–$A^2$–[$Z^2$–$A^3$]$_m$–$R^2$ I
worin $R^1$ und $R^2$ jeweils eine Alkylgruppe mit 1 bis 12 C-Atomen, worin auch eine oder zwei nicht benachbarte $CH_2$-Gruppen durch –O–, –CO–, –O–CO–, –CO–O– oder –CH=CH– ersetzt sein können, einer der Reste $R^1$ und $R^2$ auch H, F, Cl, Br oder CN,
Pyr Pyrimidin-2,5-diyl,
$A^1$ und $A^3$ jeweils trans-1,4-Cyclohexylen oder unsubstituiertes oder durch ein oder zwei F- und/oder Cl-Atome und/oder $CH_3$- und/oder CN-Gruppen substituiertes 1,4-Phenylen,
$A^2$ unsubstituiertes oder durch ein oder zwei F- und/oder Cl-Atome und/oder $CH_3$- und/oder CN-Gruppen substituiertes 1,4-Phenylen,
$Z^1$ –$CH_2CH_2$–, –$CH_2O$–, oder –$OCH_2$–
$Z^2$ –CO–O–, –O–CO–, –$CH_2CH_2$–
–$CH_2O$–, –O–$CH_2$– oder eine Einfachbindung, und,
m 0 oder 1 bedeuten.
2. Pyrimidinderivate nach Anspruch 1 gekennzeichnet durch die Teilformeln Ia und Ic:
$R^1$–Pyr–Phe–$Z^1$–Phe–$R^2$ Ia

R¹–Pyr-Phe–Z¹–Phe–Z²–A³–R² Ic

worin Phe eine 1,4-Phenylengruppe bedeutet, die gegebenenfalls auch durch ein oder zwei F- und/oder Cl-Atome und/oder CH₃- und/oder CN-Gruppe substituiert sein kann, und R¹, R², Z¹, Z², A³ und Pyr die in Anspruch 1 angegebene Bedeutung haben.

3. Pyrimidinderivate nach Anspruch 2 gekennzeichnet durch die Teilformeln Iaa und Iab:

R¹–Pyr–Phe–OCH₂–Phe–R² Iaa

R¹–Pyr–Phe–CH₂CH₂–Phe–R² Iab

worin R¹, R², Pyr und Phe die angegebene Bedeutung haben.

4. Pyrimidinderivate nach einem der Ansprüche 1 bis 3, dadurch gekennzeichnet, daß A² 1,4-Phenylen oder 2- bzw. 3-Fluor-1,4-phenylen bedeutet.

5. Pyrimidinderivate nach Anspruch 1, dadurch gekennzeichnet, daß m 0 und Z² eine Einfachbindung bedeutet.

6. Pyrimidinderivate nach Anspruch 1, dadurch gekennzeichnet, daß m 1, Z² eine Einfachbindung und A³ trans-1,4-Cyclohexylen ist.

7. Pyrimidinderivate nach mindestens einem der Ansprüche 1 bis 6, dadurch gekennzeichnet, daß R¹ und R² jeweils geradkettiges Alkyl, Alkoxy oder Oxaalkyl mit 1 bis 12 C-Atomen oder R² auch F, Cl, Br oder CN bedeuten.

8. Pyrimidinderivate nach Anspruch 1, dadurch gekennzeichnet, daß sie eine verzweigte Flügelgruppe R¹ bzw. R² tragen.

9. Pyrimidinderivate nach mindestens einem der Ansprüche 1 bis 8, dadurch gekennzeichnet, daß Pyr in 5-Position mit R1 verknüpft ist.

10. Verfahren zur Herstellung von Pyrimidinderivaten nach Anspruch 1, dadurch gekennzeichnet, daß man eine Verbindung, die sonst der Formel I entspricht, aber anstelle von H-Atomen eine oder mehrere reduzierbare Gruppe(n) und/oder zusätzliche C–C-Bindung(en) enthält, mit einem reduzierenden Mittel behandelt, oder daß man zur Herstellung von Ethern der Formel I eine entsprechende Hydroxyverbindung verethert.

11. Verwendung der Pyrimidinderivate nach Anspruch 1 als Komponenten flüssigkristalliner Phasen für Displays, die auf dem Prinzip der verdrillten Zelle (TN-Displays) beruhen.

12. Flüssigkristalline Phase mit mindestens zwei flüssigkristallinen Komponenten, dadurch gekennzeichnet, daß sie mindestens eine Verbindung der Formel I nach Anspruch 1 enthält.

13. Flüssigkristallanzeigeelement beruhend auf dem Prinzip der verdrillten Zelle (TN-Display), dadurch gekennzeichnet, daß es eine Phase nach Anspruch 12 enthält.

14. Elektrooptisches Anzeigeelement beruhend auf dem Prinzip der verdrillten Zelle (TN-Display) nach Anspruch 13, dadurch gekennzeichnet, daß es eine Phase nach Anspruch 12 enthält.

## Claims

1. Pyrimidine derivatives of the formula I

R¹–Pyr–A¹–Z¹–A²–[Z²–A³]ₘ–R² I in which R¹ and R² each are an alkyl group having 1 to 12 C atoms, in which one or two non-adjacent CH₂ groups can also be replaced by –O–, –CO–, –O–CO–, –CO–O– or –CH=CH–, and one of the radicals R¹ and R² are H, F, Cl, Br or CN,

Pyr is pyrimidine-2,5-diyl,

A¹ and A³ each are trans-1,4-cyclohexylene, unsubstituted 1,4-phenylene or 1,4-phenylene which is substituted by one or two F and/or Cl atoms and/or CH₃ and/or CN groups,

A² is 1,4-phenylene which is unsubstituted or substituted by one or two F and/or Cl atoms and/or CH₃ and/or CN groups, Z¹ is –CH₂CH₂–, –CH₂O–, or –OCH₂–

Z² is –CO–O–, –O–CO–, –CH₂CH₂–, –CH₂O–, –O–CH₂– or a single bond, and m is 0 or 1.

2. Pyrimidine derivatives according to claim 1 of the partial formulae Ia and Ic:

R¹–Pyr–Phe–Z¹–Phe–R² Ia

R¹–Pyr–Phe–Z¹–Phe–Z²–A³–R² Ic in which Phe is 1,4-phenylene which, if desired, can also be substituted by one or two F- and/or Cl-atoms and/or CH₃ and/or CN groups, and R¹, R², Z¹, Z², A³ and Pyr have the meaning specified in claim 1.

3. Pyrimidine derivatives according to claim 2 of the partial formulae Iaa and Iab:

R¹–Pyr–Phe–OCH₂–Phe–R² Iaa

R¹–Pyr–Phe–CH₂CH₂–Phe–R² Iab

in which R¹, R², Pyr and Phe have the specified meaning.

4. Pyrimidine derivatives according to one of the claims 1 to 3 characterised in that A² is 1,4-Phenylen or 2- or 3-fluor-1,4-phenylen.

5. Pyrimidine derivatives according to claim 1 characterised in that m is 0 and Z² is a single bond.

6. Pyrimidine derivatives according to claim 1 characterised in that m is 1, Z² is a single bond and A³ is trans-1,4-cyclohexylene.

7. Pyrimidine derivatives according to at least one of the claims 1 to 6 characterised in that $R^1$ and $R^2$ each are straight-chain alkyl, alkoxy or oxaalkyl having 1 to 12 C atoms and $R^2$ also is F, Cl, Br or CN.

8. Pyrimidine derivatives according to claim 1 characterised in that they have a branched wing-group $R^1$ or $R^2$.

9. Pyrimidine derivatives according to at least one of the claims 1 to 8 characterised in that Pyr is attached to $R^1$ in 5-position.

10. Process for preparing pyrimidine derivatives according to claim 1, characterised in that a compound which otherwise conforms to the formula I but contains in place of H atoms one more reducible group and/or additional C–C bonds is treated with a reducing agent, or in that, to prepare ethers of the formula I, a corresponding hydroxy compound is etherified.

11. Use of the pyrimidine derivatives according to claim 1, as components of liquid crystal phases for displays which are based on the principle of the twisted cell (TN displays).

12. Liquid crystal phase comprising at least two liquid crystal components, characterised in that it contains at least one compound of the formula I according to claim 1.

13. Liquid crystal display element which is based on the principle of the twisted cell (TN-display), characterised in that it contains a phase according to claim 12.

14. Electro-optical display element which is based on the principle of the twisted cell (TN-display) according to claim 13, characterised in that it contains a phase according to claim 12.

**Revendications**

1. Dérivés de la pyrimidine répondant à la formule I
$R^1$–Pyr–$A^1$–$Z^1$–$A^2$–$[Z^2$–$A^3]_m$–$R^2$  I
dans laquelle $R^1$ et $R^2$ représentent chacun un groupe alkyle en C1–C12 dans lequel un ou deux groupes $CH_2$ non voisins peuvent être remplacés par –O–, –CO–, –O–CO–, –CO–O– ou –CH=CH–, l'un des symboles $R^1$ et $R^2$ pouvant également représenter H, F, Cl, Br ou CN,
Pyr représente le groupe pyrimidine-2,5-diyle,
$A^1$ et $A^3$ représentent chacun le groupe trans-1,4-cyclohexylène ou le groupe 1,4-phénylène non substitué ou substitué par un ou deux atomes de F et/ou Cl et/ou groupes $CH_3$ et/ou CN,
$A^2$ représente le groupe 1,4-phénylène non substitué ou substitué par un ou deux atomes de F et/ou de Cl et/ou groupes $CH_3$ et/ou CN,
$Z^1$ représente –$CH_2CH_2$–, –$CH_2O$– ou –O–$CH_2$–,
$Z^2$ représente –CO–O–, –O–CO–, –$CH_2CH_2$–, –$CH_2O$–, –O–$CH_2$– ou une liaison simple, et
m est égal à 0 ou 1.

2. Dérivés de la pyrimidine selon la revendication 1, caractérisés par les formules partielles Ia et Ic
$R^1$–Pyr–Phe–$Z^1$–Phe–$R^2$  Ia
$R^1$–Pyr–Phe–$Z^2$–$A^3$–$R^2$  Ic
dans lesquelles Phe représente un groupe 1,4-phénylène qui peut le cas échéant être substitué par un ou deux atomes de F et/ou de Cl et/ou groupes $CH_3$ et/ou CN, et $R^1$, $R^2$, $Z^1$, $Z^2$, $A^3$ et Pyr ont les significations indiquées dans la revendication 1.

3. Dérivés de la pyrimidine selon la revendication 2, caractérisés par les formules partielles Iaa et Iab
$R^1$–Pyr–Phe–$OCH_2$–Phe–$R^2$  Iaa
$R^1$–Pyr–Phe–$CH_2CH_2$–Phe–$R^2$  Iab
dans lesquelles $R^1$, $R^2$, Pyr et Phe ont les significations indiquées ci-dessus.

4. Dérivés de la pyrimidine selon une des revendications 1 à 3, caractérisés en ce que $A^2$ représente un groupe 1,4-phénylène ou 2- ou 3-fluoro-1,4-phénylène.

5. Dérivés de la pyrimidine selon la revendication 1, caractérisés en ce que m est égal à 0 et $Z^2$ représente une liaison simple.

6. Dérivés de la pyrimidine selon la revendication 1, caractérisés en ce que m est égal à 1, $Z^2$ représente une liaison simple et $A^3$ un groupe trans-1,4-cyclohexylène.

7. Dérivés de la pyrimidine selon l'une au moins des revendications 1 à 6, caractérisés en ce que $R^1$ et $R^2$ représentent chacun un groupe alkyle, alcoxy ou oxaalkyle à chaîne droite en C1–C12, ou bien $R^2$ représente F, Cl, Br ou CN.

8. Dérivés de la pyrimidine selon la revendication 1, caractérisés en ce qu'ils portent un groupe d'extrémité $R^1$ ou $R^2$ ramifié.

9. Dérivés de la pyrimidine selon l'une au moins des revendications 1 à 8, caractérisés en ce que le groupe Pyr est relié en position 5 avec $R^1$.

10. Procédé de préparation des dérivés de la pyrimidine selon la revendication 1, caractérisé en ce que l'on traite par un agent réducteur un composé répondant par ailleurs à la formule I mais contenant, à la place d'atomes d'hydrogène, un ou plusieurs groupes réductibles et/ou en outre une ou plusieurs liaisons C–C, ou bien, pour la préparation des éthers de formule I, on éthérifie un composé hydroxylé correspondant.

11. Utilisation des dérivés de la pyrimidine selon la revendication 1 en tant que composants de phases à cristaux liquides pour des affichages basés sur le principe de la cellule torsadée (affichages TN).

12. Phase à cristaux liquides contenant au moins deux composants à cristaux liquides, caractérisée en ce qu'elle contient au moins un composé de formule I selon la revendication 1.

13. Elément d'affichage à cristaux liquides basé sur le principe de la cellule torsadée (affichage TN), caractérisé en ce qu'il contient une phase selon la revendication 12.

14. Elément d'affichage électro-optique basé sur le principe de la cellule torsadée (affichage TN) selon la revendication 13, caractérisé en ce qu'il contient une phase selon la revendication 12.